# EUROPEAN PATENT SPECIFICATION

(11) **EP 0 624 422 B1**
(45) Date of publication and mention of the grant of the patent: **08.07.1998**
(21) Application number: 94302512.2
(22) Date of filing: 11.04.1994
(51) Int. Cl.: B23K 26/00

(54) **Status indicator system for a laser beam**
Zustandzeigevorrichtung für einem Laserstrahl
Système indicateur d'état pour un faisceau laser

(30) Priority: 13.05.1993 US 61489
(43) Date of publication of application: 17.11.1994
(73) Proprietor: PLC MEDICAL SYSTEMS, INC., Milford, MA 01757 (US)
(72) Inventor: Rudko, Robert I., Holliston, Massachusetts 01746 (US)
(74) Representative: Jones, Graham H.

(56) References cited:
- US-A- 4 478 217
- US-A- 4 582 405
- PATENT ABSTRACTS OF JAPAN vol. 13 no. 414 (C-635) ,13 September 1989 & JP-A-01 151466 (MATSUSHITA ELECTRIC IND CO LTD) 14 June 1989,

## Description

### FIELD OF INVENTION

This invention relates to an improved surgical laser system with a status indicator system for indicating when a laser beam, especially an invisible one, is ready to fire or not ready to fire.

### BACKGROUND OF INVENTION

Lasers are becoming an ever more important part of the surgical environment. They provide a precision, sterile means for performing even the most intricate of procedures. In return they require intense concentration and full attention of the operator and staff. Typically surgical laser equipment uses a CO₂ laser or other laser whose beam is not readily visible to the human eye but is nonetheless dangerous. To partially address this problem visible non-harmful aiming beams such as from red HeNe lasers are provided coincident with the track of the main beam to visually illustrated the path of the laser beam. This also aids the surgeon in aiming the device so that it is perfectly aimed at the surgical site when the main laser beam is energized. In addition, indicators are employed on the laser equipment to show when the main beam is energized. However, there are still a number of problems that occur. When a surgeon is aiming the laser using a microscope or a laparoscope or in any other manner he must pay careful, continuous attention to the site and the laser beam. But at some point when he is ready to operate he must check to see if the laser is energized. This requires him to look up out of the target field of view and momentarily lose sight of the target and perhaps lose the registration of the beam. In addition, since the handpiece is separated from the laser and the main housing by the articulated arm that carries the beam from the laser to the handpiece, the surgeon or others may pick up or handle the handpiece without being aware that the laser is energized. Adding more indicators at the handpiece is expensive and complicated since the indicator has to be wired through the articulated arm. It is also cumbersome because the surgeon has to hold the handpiece for precise manipulation and may cover the indicator. Further, the indicator, even the whole handpiece, may not be visible in the surgeon's field of view. JP-A-1-151466 discloses a laser treatment apparatus including an invisible laser beam and a LED visible light. When the laser beam is energized, then also the led is energized, providing a visible signal that the laser beam is on. US-A-4478217 discloses a handpiece which vibrates when the laser beam is firing. US-A-4582405 discloses an ophthalmological laser system in which a pulsed surgical Nd:YAG laser beam is directed within a continuous visible He-Ne focusing beam and both beams are focused at the same point.

### SUMMARY OF INVENTION

It is therefore an object of this invention to provide an improved status indicator for alerting users that a laser beam is energized.

It is a further object of this invention to provide such an improved status indicator which alerts a user to the status of the laser beam without requiring the user to look away from the target field of view.

It is a further object of this invention to provide such an improved status indicator which requires no additional indicators on the handpiece or anywhere else in the system.

It is a further object of this invention to provide such an improved status indicator which continuously displays the status of the laser beam.

It is a further object of this invention to provide such a handpiece which is particularly advantageous with use for surgical lasers and any other lasers whose beams are invisible.

The invention, which is defined by claim 1, results from the realization that a truly simple yet extremely effective indicator of the energization state of a laser beam can be achieved by modifying a harmless aiming beam or a separate status beam so that the state of that beam: steady or fluctuating, red or green, broad or narrow, represents the condition of the main laser beam.

This invention features a status indicator system for a laser beam including a laser and means for transporting the laser beam from a laser to a target. There is an illumination source for providing a visible status beam through the means for transporting with the laser beam to the target. Switching means switch the laser between energized and unenergized conditions and correspondingly switch the status beam between different states, one associated with each of the conditions of the laser.

In a preferred embodiment there are only two different states of the status beam: for example, two different colors, steady and fluctuating, bright and dark, broad and narrow. The laser may produce an invisible beam and may in fact be a CO₂ laser. The means for transporting may include an optical system in an articulated arm and it may further include a handpiece. The laser beam and the status beam may be generally coincident upon exiting the means for transporting. They may in fact be coaxial and the status beam may act as an aiming beam. The illumination source may itself include a second laser which provides a visible beam. That second laser may be a helium neon laser. The switching means may include a power source for driving the laser and it may include means for attenuating the output power of the illumination source beam or means for introducing a fluctuation in the intensity of the illumination source beam, or means for changing the color of the illumination source beam, or means for changing the diameter or breadth of the illumination source beam.

### DISCLOSURE OF PREFERRED EMBODIMENT

Other objects, features and advantages will occur to those skilled in the art from the following description of a preferred embodiment and the accompanying drawings, in which:
Fig. 1 is a three-dimensional view of a CO₂ surgical laser system employing the status indicator system according to this invention;
Fig. 2 is an enlarged view of the handpiece of Fig. 1 and a portion of the articulated optical arm which carries it;
Fig. 3 is a more detailed block diagram of the surgical laser system of Fig. 1 showing the status indicator system according to this invention;
Fig. 4 is a more detailed diagram of the aiming/status laser of Fig. 3 in which the status change commensurate with the condition of the CO₂ laser is a change in color;
Fig. 5 is a view similar to Fig. 4 where the change is between the steady state and fluctuating state of the status beam;
Fig. 6 is a view similar to Fig. 5 in which the change in the status beam is a change in diameter; and
Fig. 7 is a view similar to Fig. 6 in which the change in the status beam is one of intensity.

This invention may be accomplished in a status indicator system for a laser beam in which a CO₂ laser such as used in surgical laser systems can be energized in a number of different conditions: off, on, stand-by or ready. There is some means for transporting the laser beam from the laser to a target. The target typically is some body part, for example the heart, of a surgical patient. The means for transporting is likely an articulated arm which includes an optical system for propagating the laser beam from the laser output to a handpiece at the distal end which the surgeon manipulates to effect the laser surgery. This invention also includes an illumination source in addition to the primary surgical laser, which illumination source provides a visible status beam which accompanies the primary surgical laser beam through the articulated arm and out the handpiece to the target. This second illumination source typically is another laser which produces a visible and relatively harmless beam, such as a helium neon (HeNe) laser which produces a red beam used as an aiming beam for the main CO₂ surgical laser beam. Thus the surgeon sets up the entire system using the helium neon aiming beam, and then when he is ready to perform the surgery the CO₂ beam is activated and tracks along the same path to the target site as previously indicated by the helium neon red aiming beam.

In furtherance of this invention, there is some switching means which switches the laser between energized and unenergized conditions, for example from the off to a stand-by to a ready, or fully on, condition. The switching means also correspondingly switches the status beam between different states, one associated with each of the conditions of the laser. According to the invention, the switching means can switch the status beam at least between two states, one of which is associated with the ready condition and the other with the standby condition. For example, the status beam may be red and steady when the laser beam is on stand-by and it may be fluctuating red when the laser beam is ready. The status beam could also change from narrow to broad in width, or from bright to dull in intensity, to indicate the conditions of the primary surgical laser beam.

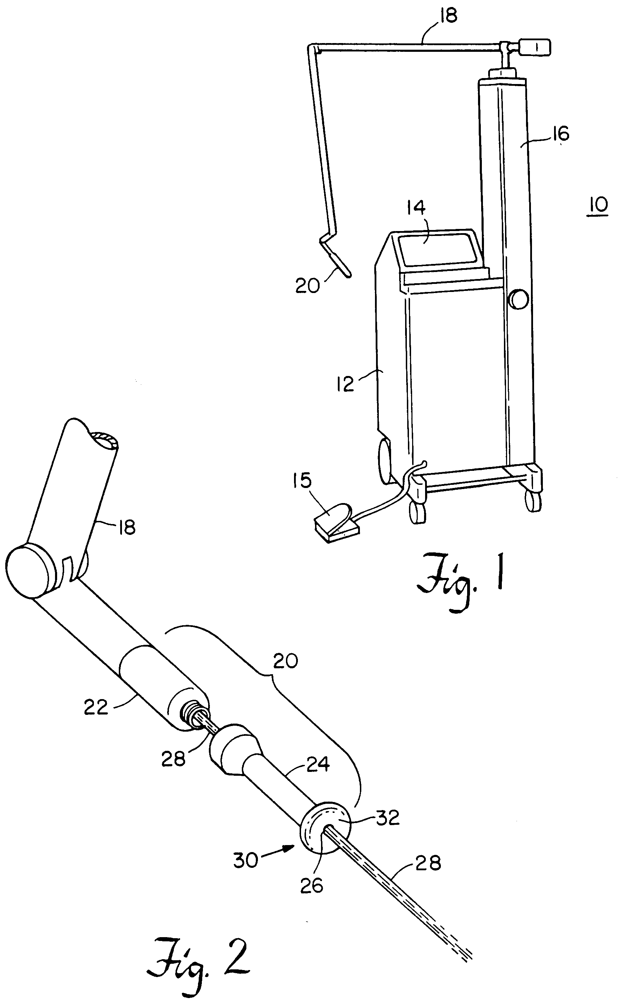

There is shown in Fig. 1 a surgical laser system 10 which includes a main housing 12 that holds the power supplies, microprocessors, computers, and other control and logic circuits that operate the system. There is a control panel 14 for operating the system. After the system is set up using control panel 14, the surgeon applies the final input to fire the laser by means of foot switch 15. Housing 16 holds the main surgical CO₂ laser and provides the laser output to articulated arm 18, which terminates in handpiece 20.

Handpiece 20, Fig. 2, may include a lens unit 22 including a lens for focusing a laser beam and a barrel 24 which includes an aperture 26 through which laser beam 28 exits. The distal end of barrel 24 includes enlarged contact surface 32 for contacting the area of the patient to be struck by the laser beam.

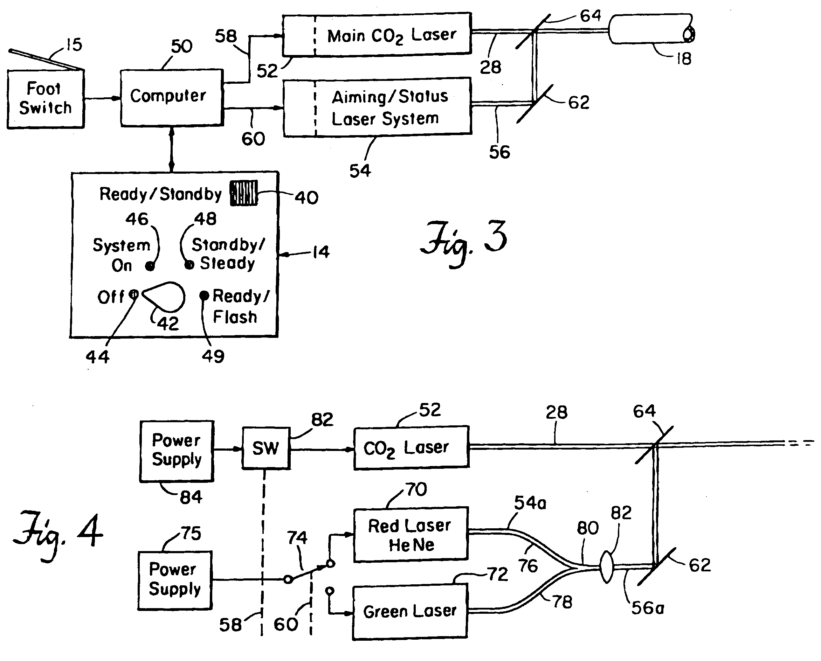

Control panel 14, Fig.3, may include a warning light 40, which for example is illuminated when the primary surgical CO₂ laser is in the ready condition, that is, will fire upon operation of foot switch 15, and may be off or displaying a different color when the CO₂ laser is in stand-by condition or when it is in the off condition. A main control switch 42 can turn the entire system off in the first position 44 or can turn the entire system on in the second position 46. Beyond that, switch 42 can set the main CO₂ surgical laser to the stand-by condition at position 48 and at the same time set the status laser to a steady red beam. In the final position 49, switch 52 can turn the main CO₂ laser to the ready condition and cause the status beam to fluctuate or flash. When the main CO₂ laser is in the ready condition it will fire upon depression of foot switch 15 by the operating surgeon. The commands from control panel 14 and foot switch 15 are employed by computer 50 to operate the main CO₂ laser 52 and the illumination source 54, which in this case is a helium neon laser that provides a red output beam 56 which also functions as an aiming beam.

The outputs 58 and 60 from computer 50 controls the operation of lasers 52 and 54, respectively. The output beam 56 from laser 54 is directed by mirror 62 to beam splitter 64, where it is joined with or becomes more or less coincident with CO₂ laser beam 28 and is directed through articulated arm 18 to handpiece 20.

For a two-condition laser beam, aiming status laser system 54a, Fig.4, may include a red helium neon laser 70, and a green helium neon laser 72. The control signal on line 60 from computer 50 then operates switch 74 which connects power supply 75 to red laser 70 in one condition, for example, the ready condition, of the CO₂ laser beam, and to green laser 72 in the stand-by condition of the CO₂ laser beam. The activated one of lasers 70 or 72 provides an output on its associated fiber optic element 76, 78. respectively, to Y junction 80 which interfaces with optics 82 to provide the output beam 56a. By stepping on foot switch 15, while the laser condition is in the ready state, and the red laser 70 is emitted as output beam 56a, the control signal on line 58 operates switch 82 to connect power supply 84 to the main laser 52.

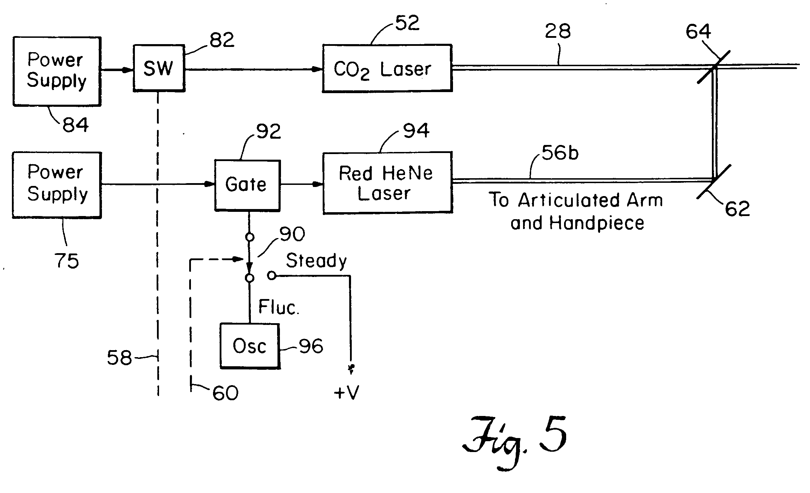

Alternatively, control signal on line 60 from computer 50 may operate switch 90, Fig. 5, which selects the input to gate 92. Gate 92 controls the input from power supply 75 to red status laser 94. When switch 90 is in the position shown, the enabling signal to gate 92 is derived from oscillator 96. Thus gate 92 alternately opens and closes so that the power applied to laser 94 is periodic instead of steady, and fluctuating bean 56b is emitted. When switch 90 is moved to the other position by the signal on line 60, then a steady input V+ is applied to gate 92 and the output from red laser 94 will be a steady beam 56b.

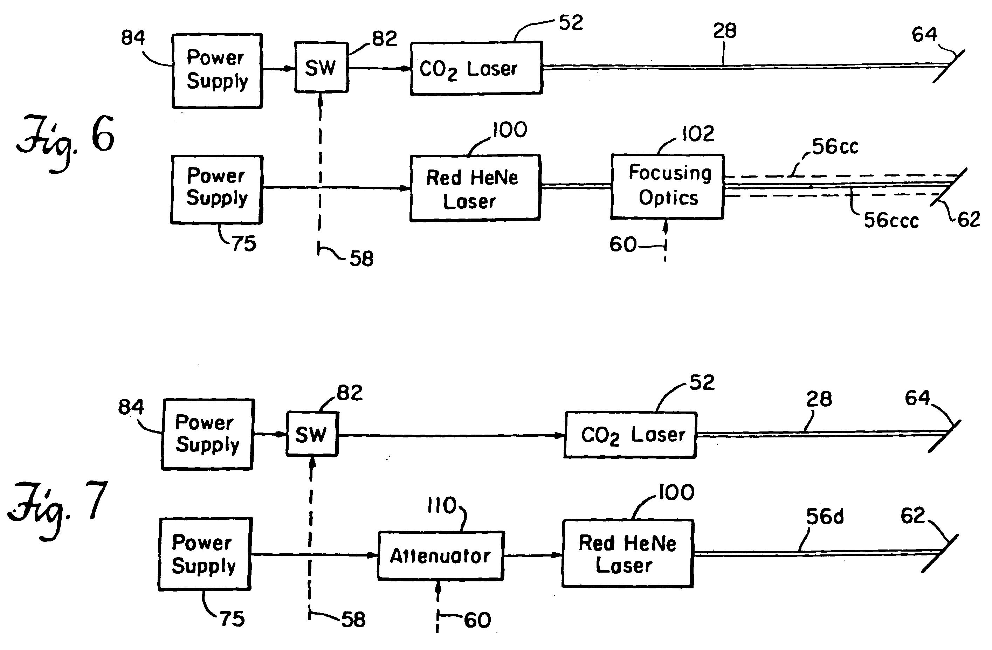

In another construction, status beam 56c, Fig. 6, derived from red helium neon laser 100, may be caused to be broadened as indicated at 56cc or narrowed as indicated at 56ccc: focusing optics 102 intercept beam 56c coming from laser 100 and in response to control signal on line 60 either broadens or narrows beam 56c. The same red helium neon laser, or any other laser which will provide a good visible marker or tracer beam, can be used in conjunction with attenuator 110, Fig. 7, which can be driven by control signal on line 60 from computer 50 to decrease the intensity of the emitted beam 56d so that the beam can have a bright and dull intensity, or even three or more levels of intensity which indicate the different levels of the main surgical CO₂ laser beam. The attenuation need not take place in a special attenuator circuit as indicated by attenuator 110; control signal on line 60 could directly affect power supply 75 or an optical attenuator could be used in the output of laser 100.

While this invention has been described with respect to its preferred embodiments, it will be understood that various modifications and variations falling within the scope of the claims will occur to those skilled in the art.

## Claims

1. A surgical laser system with a status indicator system comprising:
a surgical laser (52) which provides a surgical laser beam for surgery;
switching means (42), remote from the surgical laser (52) and having at least two switch positions, a ready position (50) for switching the surgical laser to a condition wherein the surgical laser is ready to fire and standby position (48) for switching the surgical laser to a condition wherein the surgical laser is not ready to fire;
an illumination source (54) separate from the surgical laser (52) for providing a visible laser status beam proximate the surgical laser beam, said visible laser status beam being capable of at least two states, a first state indicating that the surgical laser is ready to fire and `a second state indicating that the surgical laser is not ready to fire;
control means (50), responsive to said switching means (42) and coupled to said illumination source (54), for automatically causing said illumination source (54) to enter the first state when said switching means (42) is switched to the ready position (49), and for causing said illumination source (54) to enter the second state when said switching means is switched to the standby position (48).

2. The system of claim 1 in which said surgical laser (52) is a CO₂ laser.

3. The system of claim 1 in which said illumination source (54) is a HeNe laser.

4. The system of claim 1 in which said illumination source (54) includes a power source (75) and said control means includes means (90,92,96) for varying the amount of power supplied by said power source to fluctuate said visible laser status beam.

5. The system of claim 4 in which said control means includes means for varying the amount of power supplied by the power source (75) when said switching means (42) is in said ready position (50).

6. The system of claim 4 in which said control means includes means for varying the amount of power supplied by the power source (75) when said switching means (42) is in said standby position (48).

7. The system of claim 1 in which said illumination source includes a first laser (72) for providing a first color visible laser status beam and a second laser (70) for providing a second color visible laser status beam.

8. The system of claim 7 in which said control means includes means (74) for causing said illumination source to operate said first laser (72) when said switching means (42) is in said standby position and for causing said illumination source to operate said second laser (70) when said switching means (42) is in said ready position.

9. The system of claim 1 further including means (102) for varying the diameter of said visible laser status beam between first and second diameter beams.

10. The system of claim 9 in which said first state is the first diameter beam and said second state is the second diameter beam.

11. The system of claim 1 further including a foot switch (15) connected to said switching means (42).

12. The system of claim 11 in which said switching means includes means for operating said surgical laser to fire only when said foot switch (15) is depressed and said switching means (42 is in the ready position (49).

13. The system of any preceding claims in which said surgical laser (52) produces an invisible beam.

## Patentansprüche

1. Ein chirurgisches Lasersystem mit einer Zustandzeigevorrichtung, aufweisend:
Einen chirurgischen Laser (52), der einen chirurgischen Laserstrahl für die Chirurgie liefert;
Schalteinrichtungen (42), entfernt von dem chirurgischen Laser (52) und mit mindestens zwei Schaltstellungen, einer Bereit-Stellung (49) um den chirurgischen Laser in eine Verfassung umzuschalten, in der der chirurgische Laser bereit ist zu feuern, und einer Warte-Stellung (48) um den chirurgischen Laser in eine Verfassung umzuschalten, in der der chirurgische Laser nicht bereit ist zu feuern;
Eine Beleuchtungsquelle (54), die getrennt von dem chirurgischen Laser (52) ist, um einen sichtbaren Laserzustandsstrahl nahe dem chirurgischen Laserstrahl zu schaffen, wobei besagter sichtbarer Laserzustandsstrahl zumindest zweier Zustände fähig ist, eines ersten Zustands, der anzeigt, daß der chirurgische Laser bereit ist zu feuern, und eines zweiten Zustands, der anzeigt, daß der chirurgische Laser nicht bereit ist zu feuern;
Steuereinrichtungen (50), die auf besagte Schalteinrichtungen (42) ansprechen und an besagte Beleuchtungsquelle (54) angeschlossen sind, um besagte Beleuchtungsquelle (54) automatisch zu veranlassen, in den ersten Zustand einzutreten, wenn besagte Schalteinrichtungen (42) in die Bereit-Stellung (49) geschaltet werden, und um besagte Beleuchtungsquelle (54) zu veranlassen, in den zweiten Zustand einzutreten wenn besagte Schalteinrichtungen in die Warte-Stellung (48) geschaltet werden.

2. Das System des Anspruchs 1, in dem besagter chirurgischer Laser (52) ein CO₂-Laser ist.

3. Das System des Anspruchs 1, in dem besagte Beleuchtungsquelle (54) ein HeNe-Laser ist.

4. Das System des Anspruchs 1, in dem besagte Beleuchtungsquelle (54) eine Energiequelle (75) aufweist und besagte Steuereinrichtungen Einrichtungen (90, 92, 96) zum Ändern der Menge an Energie, die durch besagte Energiequelle geliefert wird, um besagten sichtbaren Laserzustandsstrahl fluktuierend zu machen, aufweisen.

5. Das System des Anspruchs 4, in dem besagte Steuereinrichtungen Einrichtungen zum Ändern der Menge an Energie, die durch die Energiequelle (75) geliefert wird, wenn besagte Schalteinrichtungen (42) sich in besagter Bereit-Stellung (49) befinden, aufweisen.

6. Das System des Anspruchs 4, in dem besagte Steuereinrichtungen Einrichtungen zum Ändern der Menge an Energie, die durch die Energiequelle (75) geliefert wird, wenn besagte Schalteinrichtungen (42) sich in besagter Warte-Stellung (48) befinden, aufweisen.

7. Das System des Anspruchs 1, in dem besagte Beleuchtungsquelle einen ersten Laser (72) zum Liefern eines sichtbaren Laserzustandsstrahls einer ersten Farbe und einen zweiten Laser (70) zum Liefern eines sichtbaren Laserzustandsstrahls einer zweiten Farbe aufweist.

8. Das System des Anspruchs 7, in dem besagte Steuereinrichtungen Einrichtungen (74), um besagte Beleuchtungsquelle zu veranlassen, besagten ersten Laser (72) zu betätigen, wenn besagte Schalteinrichtungen (42) sich in besagter Warte-Stellung befinden, und um besagte Beleuchtungsquelle zu veranlassen, besagten zweiten Laser (70) zu betätigen, wenn besagte Schalteinrichtungen (42) sich in besagter Bereit-Stellung befinden, aufweisen.

9. Das System des Anspruchs 1, weiter aufweisend Einrichtungen (102) zum Ändern des Durchmessers besagten sichtbaren Laserzustandsstrahls zwischen Strahlen eines ersten und eines zweiten Durchmessers zu ändern.

10. Das System des Anspruchs 9, in dem besagter erster Zustand der Strahl des ersten Durchmessers und besagter zweiter Zustand der Strahl des zweiten Durchmessers ist.

11. Das System des Anspruchs 1, weiter aufweisend einen Fußschalter (15), der mit besagten Schalteinrichtungen (42) verbunden ist.

12. Das System des Anspruchs 11, in dem besagte Schalteinrichtungen Einrichtungen zum Betätigen besagten chirurgischen Lasers derart, daß er nur feuert, wenn besagter Fußschalter (15) niedergedrückt ist und besagte Schalteinrichtungen (42) sich in der Bereit-Stellung (49) befinden, aufweisen.

13. Das System irgendeines der vorhergehenden Ansprüche, in dem besagter chirurgischer Laser (52) einen unsichtbaren Strahl erzeugt.

## Revendications

1. Système à laser chirurgical comportant un système d'indication d'état comprenant :
- un laser chirurgical (52) qui fournit un faisceau laser chirurgical pour de la chirurgie,
- des moyens de commutation (42), à distance du laser chirurgical (52) et présentant au moins deux positions de commutation, une position prêt (49) pour commuter le laser chirurgical dans un état dans lequel le laser chirurgical est prêt à faire feu et une position en attente (48) pour commuter le laser chirurgical dans un état dans lequel le laser chirurgical n'est pas prêt à faire feu,
- une source d'éclairage (54) séparée du laser chirurgical (52), pour fournir un faisceau d'état visible du laser à proximité du faisceau laser chirurgical, le faisceau d'état visible du laser pouvant avoir au moins deux états, un premier état indiquant que le laser chirurgical est prêt à faire feu et un second état indiquant que le laser chirurgical n'est pas prêt à faire feu,
- des moyens de commande (50) qui répondent aux moyens de commutation (42) et qui sont raccordés à la source d'éclairage (54), en vue d'amener automatiquement la source d'éclairage (54) à prendre le premier état lorsque les moyens de commutation (42) sont commutés dans la position prêt (49) et pour amener la source d'éclairage (54) à prendre le second état lorsque les moyens de commutation sont commutés dans la position d'attente (48).

2. Système suivant la revendication 1, caractérisé en ce que le laser chirurgical (52) est un laser à CO₂.

3. Système suivant la revendication 1, caractérisé en ce que la source d'éclairage (54) est un laser à HeNe.

4. Système suivant la revendication 1, caractérisé en ce que la source d'éclairage (54) comprend une source d'alimentation (75) et en ce que les moyens de commande comprennent des moyens (90, 92, 96) pour modifier la quantité d'énergie fournie par la source d'alimentation pour faire fluctuer le faisceau d'état visible du laser.

5. Système suivant la revendication 4, caractérisé en ce que les moyens de commande comprennent des moyens pour modifier la quantité d'énergie fournie par la source d'alimentation (75) lorsque les moyens de commutation (42) sont dans la position prêt (49).

6. Système suivant la revendication 4, caractérisé en ce que les moyens de commande comprennent des moyens pour modifier la quantité d'énergie fournie par la source d'alimentation (75) lorsque les moyens de commutation (42) sont dans la position d'attente (48).

7. Système suivant la revendication 1, caractérisé en ce que la source d'éclairage comprend un premier laser (72) pour fournir un faisceau d'état visible du laser d'une première couleur et un second laser (70) pour fournir un faisceau d'état visible du laser d'une seconde couleur.

8. Système suivant la revendication 7, caractérisé en ce que les moyens de commande comprennent des moyens (74) pour amener la source d'éclairage à faire fonctionner le premier laser (72) lorsque les moyens de commutation (42) sont dans la position d'attente et pour amener la source d'éclairage à faire fonctionner le second laser (70) lorsque les moyens de commutation (42) sont dans la position prêt.

9. Système suivant la revendication 1, caractérisé en ce qu'il comprend en outre des moyens (102) pour modifier le diamètre du faisceau d'état visible du laser entre des faisceaux de premier et de second diamètres.

10. Système suivant la revendication 9, caractérisé en ce que le premier état cité est le faisceau de premier diamètre et en ce que le second état susdit est le faisceau de second diamètre.

11. Système suivant la revendication 1, caractérisé en ce qu'il comprend en outre un commutateur à pédale (15) raccordé au moyens de commutation (42).

12. Système suivant la revendication 11, caractérisé en ce que les moyens de commutation comprennent des moyens pour faire fonctionner le laser chirurgical afin de ne faire feu que lorsque le commutateur à pédale (15) est enfoncé et lorsque les moyens de commutation (42) sont dans la position prêt (49).

13. Système suivant l'une quelconque des revendications précédentes, caractérisé en ce que le laser chirurgical (52) produit un faisceau invisible.
